# EUROPEAN PATENT SPECIFICATION

(11) **EP 0 931 560 B1**
(45) Date of publication and mention of the grant of the patent: **15.09.2004**
(21) Application number: 98310493.6
(22) Date of filing: 21.12.1998
(51) Int. Cl.: A61M 25/02

(54) **Catheter clamps**
Katheterklemmen
Clamps pour cathéter

(30) Priority: 14.01.1998 GB 9800634; 24.02.1998 GB 9803671
(43) Date of publication of application: 28.07.1999
(73) Proprietor: Smiths Group plc, London, NW11 8DS (GB)
(72) Inventor: Batchelor, Kester Julian, Burnham-on-Sea, Somerset TA8 2RY (GB)
(74) Representative: Flint, Jonathan McNeill

(56) References cited:
- EP-A- 0 778 041
- AU-B- 589 549
- US-A- 3 568 679
- US-A- 4 517 971
- US-A- 4 659 329

## Description

This invention relates to catheter clamps of the kind having a base for attachment to the skin and an opening into the clamp through which the catheter enters the clamp.

Catheters are often fixed where they emerge from the body, such as by means of adhesive tape or a clamp fixed to the skin by an adhesive or sutures, in order to prevent the catheter being inadvertently pulled out of the body and to protect the skin entry site.

Examples of clamps for fixing a catheter are described in GB2288542; GB2288538; US4645492; GB2147811; US4261363; US4659329; US4360025; US4533349; EP327299; GB2115290; EP648512; GB2234172; "A method of securing epidural catheters" by Schmitt LG and Ullma DA, Anaest Analg 1989: 69: 856-7; "Securing epidural catheters, a modification" by BE Harrington and DJ Kopacz, Anaest Analg 1990: 71: 440-6; and "New device for the securement of epidural catheters" by SB Corn and S Datta, Regional Anaesthesia, vol 20, No 6, Nov-Dec 95, 545-546. One form of epidural catheter clamp is sold by SIMS Portex Inc of Keene, New Hampshire, USA under the trade mark "Sure Snap". EP 0778041, being the base of the preamble of claim 1 describes a stabilizing device for a catheter entering the body where the catheter is formed into a loop and is held by a clip close to the point at which it enters the body. Such a device does not allow movement of the catheter tip into the body. US 4517971 describes a guard for a venipuncture site where a catheter is wrapped around two spools. Again, such an arrangement does not permit any substantial movement of the catheter.

In some applications, such as in epidural anaesthesia, there can be appreciable relative movement between the site of the patient end of the catheter, within the body, and the site on the skin surface at which the catheter emerges from the body. In epidural anaesthesia, this relative movement is caused by sliding of tissue layers relative to one another as the patient moves. Where the catheter is not fixed at the skin, this relative movement can cause migration of the catheter into the body. This has been reported by C Hamilton and E Riley in "Changes in the position of Epidural catheters associated with Patient Movement", Anesthesiology 1997: 86: 778-84. This does not cause a significant problem where the catheter is not fixed, because the patient end of the catheter will remain in the epidural space as this moves up or down relative to the skin site. Where, however, the catheter is fixed at the skin surface, this relative movement can cause the patient end of the catheter to pull out of the epidural space, preventing the administration of anaesthetic. In order to avoid these problems, clinicians now often insert an increased length of catheter into the epidural space and secure it with adhesive tape on the skin. The problem with this is that the increased length of catheter in the epidural space makes it more likely that the patient end of the catheter will deviate from the centre line, giving rise to an increased incidence of complications such as unilateral blocks.

It is an object of the present invention to provide an improved catheter clamp and an assembly including a clamp and a catheter.

According to the present invention there is provided a catheter clamp of the above-specified kind, characterised in that the catheter can move freely through the opening, that the clamp includes a grip for retaining the catheter against movement along its length, and a region between the opening and the grip in which the catheter can be formed into a loose loop such that the catheter can move freely to a limited extent through the opening when retained at the grip.

The clamp may include a lid displaceable over the base between an open position and a closed position. The lid is preferably hinged on the base. The grip preferably applies a greater gripping force to the catheter when the lid is closed than when the lid is open. The grip may have two walls between which the catheter extends, the walls being urged closer together by engagement with the lid. The clamp may include a clip that holds the catheter in a loop when the lid is open and releases the catheter when the lid is closed. The clip preferably includes a plate having a first end that holds the catheter and a second end located for engagement by a part of the lid such that the first end releases engagement of the catheter when the second end is engaged by the lid. The base may have markings in the region to indicate the path of the loop of the catheter. The base may include a surface formation in the region on which the user holds the catheter while it is being secured with the grip. The grip preferably includes a resilient member with a channel therethrough, the catheter extending along the channel.

An epidural catheter clamp assembly according to the present invention, will now be described, by way of example, with reference to the accompanying drawings, in which:
- Figure 1: is a perspective view from one end of the clamp and catheter with the clamp open;
- Figure 2: is a plan view of the clamp and catheter;
- Figure 3: is a perspective view of the clamp from its opposite end without the catheter; and
- Figure 4: is a perspective view of an alternative clamp and catheter with the clamp open.

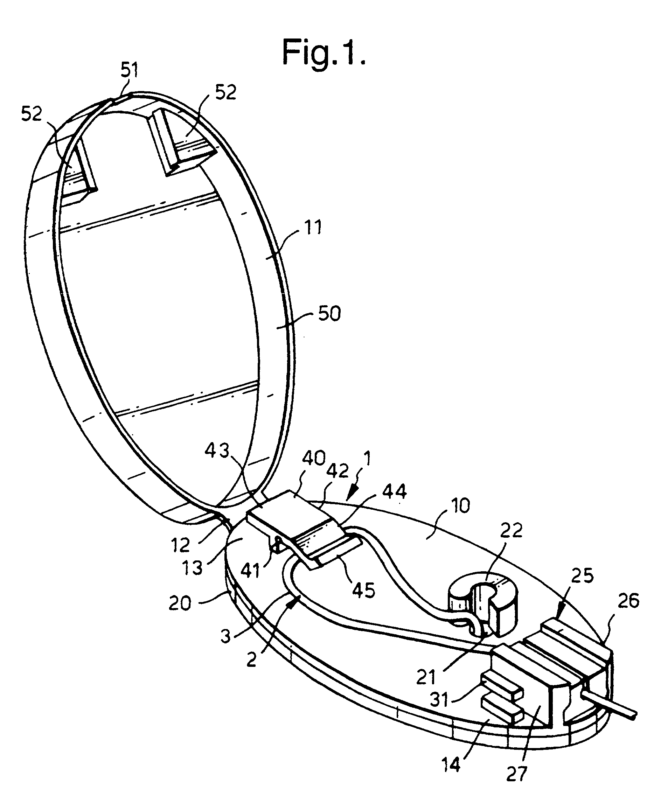

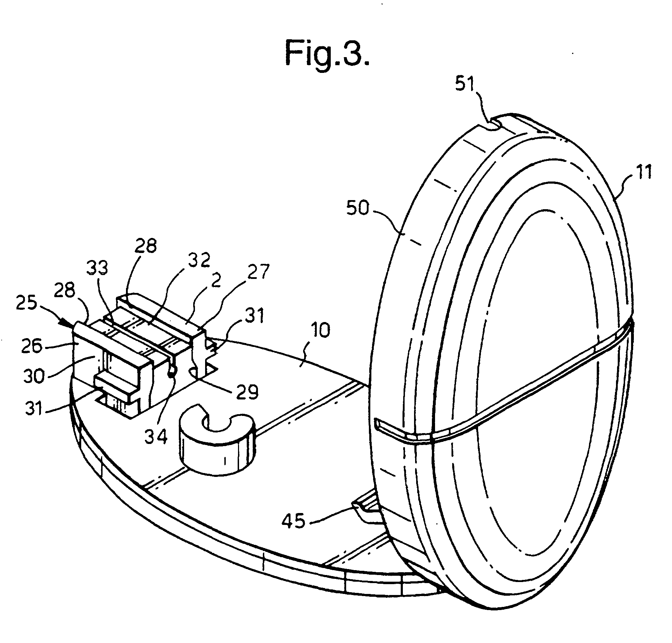

With reference first to Figures 1 to 3, the clamp includes a one-piece moulding 1 of a rigid plastics material providing an oval base 10 and a lid 11 attached to the base by a flexible web hinge 12 at the rear end 13. An annular foam pad 20 is attached to the lower surface of the base 10, the pad having a lower surface coated with a skin-compatible adhesive.

The base 10 has a small, circular opening 21 located slightly off centre from the midline of the base and towards the forward end 14, opposite the hinge 12. The opening 21 is partly surrounded by a part cylindrical wall 22 of C-shape in section. The diameter of the opening 21 is greater than that of the epidural catheter 2 so that it can move freely through the opening.

At the forward end 14, the base 10 has a grip 25 for gripping the catheter 2. The grip 25 comprises two parallel walls 26 and 27 moulded integrally with the base, which extend parallel to one another and to the midline of the base 10. The walls 26 and 27 are reduced in thickness on their inner, facing surfaces 28 so that the gap between the two walls has an enlarged portion 29 close to the base 10. On their outer surfaces 30, each wall 26 and 27 has an outwardly-projecting lip 31, the purpose of which will be explained later. The grip 25 is completed by an insert 32 of a soft, resilient rubber or similar material fitted between the walls 26 and 27 and retained by engagement in the enlarged portion 29. The insert 32 has a slot 33 formed along its upper surface and extending about half way down the height of the insert. The slot 33 is enlarged at its lower end to form a recess 34 of circular section having a diameter similar to that of the epidural catheter 2.

A catheter clip 40 is moulded integrally with the base 10 towards the centre of its rear end 13. The clip 40 comprises a short vertical stem 41 supporting a rigid horizontal plate 42 extending longitudinally of the clamp and projecting on both sides of the stem. One side 43 of the plate 42 projects rearwardly parallel to the surface of the base 10; the opposite side 44 projects forwardly and is inclined downwardly to contact the upper surface of the base, where it is formed with a short, upwardly-directed edge or lip 45. The operation of the clip 40 will be described later.

The lid 11 has the same shape as the base 10, with an outer peripheral wall 50 shaped to fit snugly about the outer edge of the base when the lid is folded down about its hinge 12. At its forward end, the wall 50 has a slot 51 aligned with the forward end of the slot 33 in the grip 25 so that the catheter 2 can extend out of the clamp when the lid 11 is closed. On its inside, close to the slot 51, the lid 11 has two catches 52 located to engage the lips 31 on the walls 26 and 27 when the lid is folded down, so as thereby to retain the lid in the closed position. The wall 50 close to the hinge 12 overhangs the rear side 43 of the clip plate 42 when the lid 11 is up or open, and, when the lid is pushed down to close the clamp, the wall contacts and depresses the rear side of the plate, thereby raising its forward end 44 as the plate flexes about the top of the stem 41.

In use, the patient end of the epidural catheter is introduced to the epidural space in the usual way, with its machine end emerging from the skin. The clamp is initially in an open state with the lid 11 up and the machine end of the epidural catheter 2 where it emerges from the skin is threaded through the aperture 21. The foam pad 20 is adhered to the skin around the site where the catheter emerges from the body. The catheter 2 is then formed into a loop 3 over the upper surface of the base 10 and is lead under the forward side 44 of the plate 42, where it is gripped between the lower edge 45 of the plate and the upper surface of the base. The catheter 2 is then led back to extend across the upper surface of the resilient insert 32 in the grip 25 and is pushed down into the enlarged recess 34 where it is held by friction with the insert. The lid 11 is then pushed down to close the clamp. As the lid 11 is pushed down, the forward end 44 of the clip 40 is lifted up, thereby releasing its engagement with the catheter 2, so that the loop 3 is now loose and free beneath the lid. Closing the lid 11 also has the effect of squeezing together the walls 26 and 27, thereby compressing the insert 32 so that it grips the catheter 2 more securely. With the lid 11 closed, the catheter 2 is only retained by the grip 25 and is free to move within the clamp and, in particular, is free to move in or out of the opening 21. The loop 3 is protected by the lid 11 from external contamination.

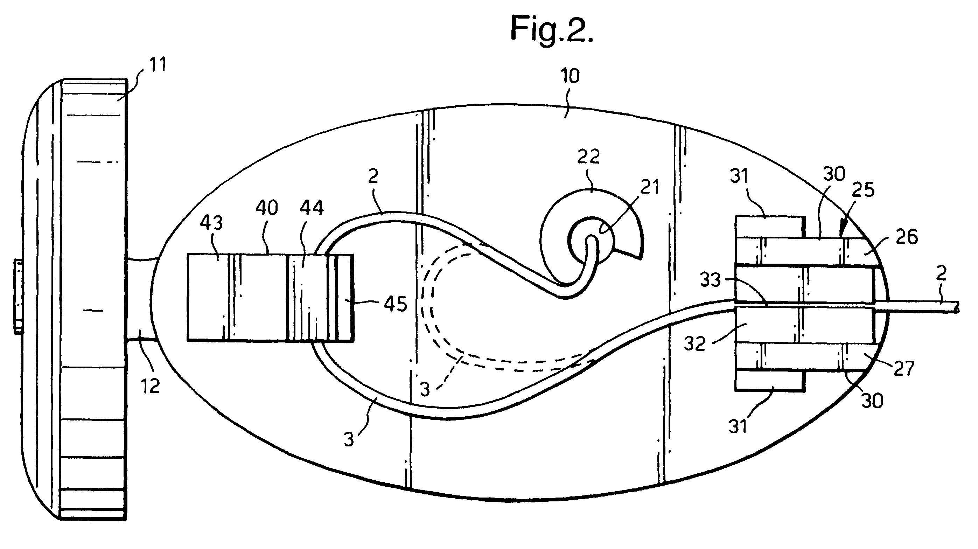

Any force exerted on the machine end of the catheter 2 outside the clamp will be resisted by the grip 25. The patient end of the catheter 2, however, is free to be displaced by a limited extent, as shown in Figure 2. The initial position of the catheter 2 is indicated by the full lines; the position after being pulled into the body is indicated by the broken lines. It can be seen that, in the latter position, the slack provided by the loop 3 of catheter 2 in the clamp allows the patient end of the catheter to be displaced freely into the body. If the catheter were instead fixed where it enters the skin, the patient end could be pulled out of the epidural space. To remove the catheter 2 from the clamp, the lid 11 is hinged up, pulling the catch 52 out of engagement with the lips 31.

The clamp of the present invention, therefore, reduces the risk of the catheter 2 being pulled out of the epidural space as a result of migration of the catheter into the body, without the need to insert an increased length of catheter. This thereby reduces the risk of unilateral block. The portion of the catheter 2 that is free to migrate into the body is protected from contamination by the clamp, as is the site of entry of the catheter into the body. This, thereby reduces the risk of infection.

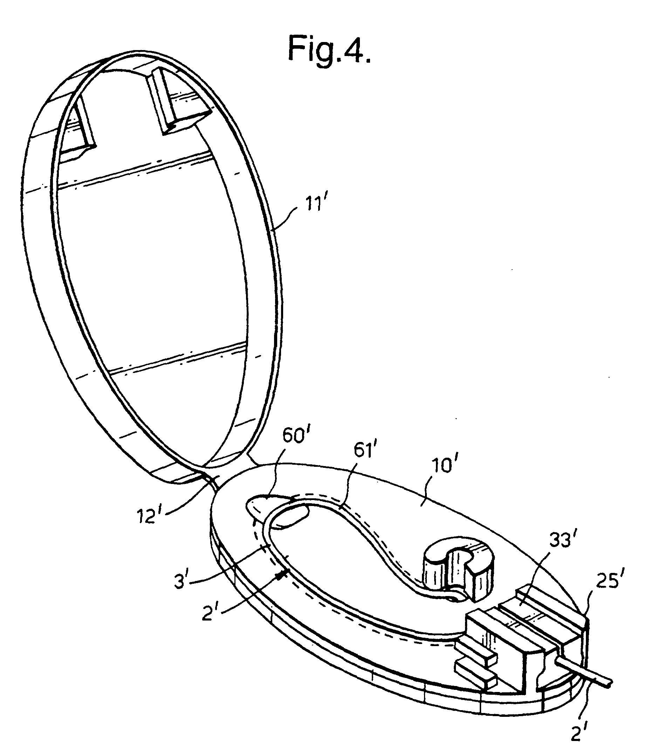

There are various modifications that could be made to the clamp. For example, as shown in Figure 4, the clip could be omitted. In this arrangement, the base 10' is formed with a recessed dimple 60' close to the hinge 12' and has markings 61' to indicate the track of the loop 3'. The user simply forms the catheter 2' manually into the loop 3' by leading along the markings 61' and holding the rear end of the loop with his finger placed on the catheter where it extends over the dimple 60'. With his other hand, the user pushes the catheter into the slot 33' in the grip 25'. The catheter is then released and the lid 11' is closed.

The clamp of the present invention is not confined to use with epidural catheters but can be used with other catheters where it is preferable that the patient end should be free to migrate into the body. There are various other ways in which the catheter could be formed into a loop in the clamp and various other ways in which it can be gripped away from the opening where the catheter enters the clamp.

## Claims

1. A catheter clamp having a base (10, 10') for attachment to the skin and an opening (21) into the clamp through which the catheter (3, 3') enters the clamp, **characterised in that** the catheter can move freely through the opening (21), that the clamp includes a grip (25, 25') for retaining the catheter (3, 3') against movement along its length and a region between the opening (21) and the grip (25, 25') in which the catheter can be formed into a loose loop (3, 3') such that the catheter can move freely to a limited extent through the opening (21) when retained at the grip (25, 25').

2. A catheter clamp according to Claim 1, **characterised in that** the clamp includes a lid (11, 11') displaceable over the base (10, 10') between an open position and a closed position.

3. A catheter clamp according to Claim 2, **characterised in that** the lid (11, 11') is hinged on the base (10, 10').

4. A catheter clamp according to Claim 2 or 3, **characterised in that** the grip (25, 25') applies a greater gripping force to the catheter (2, 2') when the lid (11, 11') is closed than when the lid is open.

5. A catheter clamp according to Claim 4, **characterised in that** the grip (25, 25') has two walls (26 and 27) between which the catheter (2, 2') extends, and that the walls (26 and 27) are urged closer together by engagement with the lid (11, 11').

6. A catheter clamp according to any one of Claims 2 to 5, including a clip (40) that holds the catheter (2) in a loop (3) when the lid (11) is open and releases the catheter when the lid is closed.

7. A catheter clamp according to Claim 6, **characterised in that** the clip (40) includes a plate (42) having a first end (45) that holds the catheter (2) and a second end (43) located for engagement by a part of the lid (11) such that the first end (45) releases engagement of the catheter (2) when the second end (43) is engaged by the lid (11).

8. A catheter clamp according to any one of Claims 1 to 5, **characterised in that** the base (10') has markings (61') in the region to indicate the path of the loop (3') of the catheter (2').

9. A catheter clamp according to any one of Claims 1 to 5 or Claim 8, **characterised in that** the base (10') includes in the region a surface formation (60') on which the user holds the catheter (2') while it is being secured with the grip (25').

10. A catheter clamp according to any one of the preceding claims, **characterised in that** the grip (25, 25') includes a resilient member (32) with a channel (33, 34) extending therethrough, and that the catheter (2, 2') extends along the channel (33, 34).

## Patentansprüche

1. Katheterklemme mit einer Basis (10, 10') zur Befestigung an der Haut und einer Öffnung (21) in der Klemme, durch die der Katheter (3, 3') in die Klemme eintritt, **dadurch gekennzeichnet, dass** der Katheter sich ungehindert durch die Öffnung (21) bewegen kann, dass die Klemme eine Halterung (25, 25') zum Festhalten des Katheters (3, 3') gegen Bewegung entlang seiner Länge und einen Bereich zwischen der Öffnung (21) und der Halterung (25, 25') beinhaltet, in welchem der Katheter in eine lose Schleife, (3, 3') geformt werden kann, so dass sich der Katheter bis zu einem begrenzten Umfang ungehindert durch die Öffnung (21) bewegen kann, wenn er an der Halterung (25, 25') festgehalten wird.

2. Katheterklemme nach Anspruch 1, **dadurch gekennzeichnet, dass** die Klemme einen Deckel (11, 11') enthält, der über die Basis (10, 10') zwischen einer offenen Stellung und einer geschlossenen Stellung versetzbar ist.

3. Katheterklemme nach Anspruch 2, **dadurch gekennzeichnet, dass** der Deckel (11, 11') an der Basis (10, 10') angelenkt ist.

4. Katheterklemme nach Anspruch 2 oder 3, **dadurch gekennzeichnet, dass** die Halterung (25, 25') eine größere Haltekraft auf den Katheter (2, 2') aufbringt, wenn der Deckel (11, 11') geschlossen ist, als wenn der Deckel geöffnet ist.

5. Katheterklemme nach Anspruch 4, **dadurch gekennzeichnet, dass** die Halterung (25, 25') zwei Wände (26 und 27) hat, zwischen denen sich der Katheter (2, 2') erstreckt, und dass die Wände (26 und 27) durch Ineingriffbringen mit dem Deckel (11, 11') näher zueinander gezwungen werden.

6. Katheterklemme nach einem der Ansprüche 2 bis 5, enthaltend einen Klipp (40), der den Katheter (2) in einer Schleife (3) hält, wenn der Deckel (11) geöffnet ist, und den Katheter freigibt, wenn der Deckel geschlossen ist.

7. Katheterklemme nach Anspruch 6, **dadurch gekennzeichnet, dass** der Klipp (40) eine Platte (42) enthält, die ein erstes Ende (45), das den Katheter (2) festhält, und ein zweites Ende (43), das zum Ineingriffbringen mit einem Teil des Deckels (11) derart angeordnet ist, dass das erste Ende (45) den Eingriff auf den Katheter (2) freigibt, wenn das zweite Ende (43) mit dem Deckel (11) in Eingriff gebracht wird, hat.

8. Katheterklemme nach einem der Ansprüche 1 bis 5, **dadurch gekennzeichnet, dass** die Basis (10') Markierungen (61') in dem Bereich hat, um den Pfad der Schleife (3') des Katheters (2') anzuzeigen.

9. Katheterklemme nach einem der Ansprüche 1 bis 5 oder Anspruch 8, **dadurch gekennzeichnet, dass** die Basis (10') in dem Bereich eine Oberflächenformung (60') enthält, auf der der Benutzer den Katheter (2') hält, während dieser durch die Halterung (25') sicher befestigt wird.

10. Katheterklemme nach einem der vorangegangenen Ansprüche, **dadurch gekennzeichnet, dass** die Halterung (25, 25') ein elastisches Teil (32) mit einem sich dadurch erstreckenden Kanal (33, 34) enthält, und dass der Katheter (2, 2') sich entlang des Kanals (33, 34) erstreckt.

## Revendications

1. Clamp pour cathéter comportant une base (10, 10') pour fixation à la peau et une ouverture (21) dans le clamp à travers laquelle le cathéter (3, 3') s'engage dans le clamp, **caractérisé en ce que** le cathéter peut se déplacer librement à travers l'ouverture (21), **en ce que** le clamp comprend une pince de serrage (25, 25') pour retenir le cathéter (3, 3') contre un mouvement le long de sa longueur, et une zone entre l'ouverture (21) et la pince de serrage (25, 25') dans laquelle le cathéter peut se mettre en boucle desserrée (3, 3') de façon que le cathéter puisse bouger librement jusqu'à une étendue limitée à travers l'ouverture (21) quand il est retenu au niveau de la pince de serrage (25, 25').

2. Clamp pour cathéter selon la revendication 1, **caractérisé en ce que** le clamp comprend un couvercle (11, 11') mobile sur la base (10, 10') entre une position ouverte et une position fermée.

3. Clamp pour cathéter selon la revendication 2, **caractérisé en ce que** le couvercle (11, 11') pivote autour de la base (10, 10').

4. Clamp pour cathéter selon la revendication 2 ou 3, **caractérisé en ce que** la pince de serrage (25, 25') applique une force de serrage plus grande au cathéter (2, 2') lorsque le couvercle (11, 11') est fermé que lorsque le couvercle est ouvert.

5. Clamp pour cathéter selon la revendication 4, **caractérisé en ce que** la pince de serrage (25, 25') comporte deux parois (26 et 27) entre lesquelles s'étend le cathéter (2, 2'), et **en ce que** les parois (26 et 27) sont pressées étroitement ensemble par emboîtement avec le couvercle (11, 11').

6. Clamp pour cathéter selon l'une quelconque des revendications 2 à 5, comprenant une bride (40) qui tient le cathéter (2) dans une boucle (3) lorsque le couvercle (11) est ouvert et libère le cathéter lorsque le couvercle est fermé.

7. Clamp pour cathéter selon la revendication 6, **caractérisé en ce que** la bride (40) comprend une plaque (42) comportant une première extrémité (45) qui tient le cathéter (2) et une seconde extrémité (43) disposée pour coopérer avec une partie du couvercle (11) de façon que la première extrémité (45) libère la prise du cathéter (2) lorsque la seconde extrémité (43) coopère avec le couvercle (11).

8. Clamp pour cathéter selon l'une quelconque des revendications 1 à 5, **caractérisé en ce que** la base (10') comporte des marques (61') dans la zone pour indiquer le chemin de la boucle (3') du cathéter (2').

9. Clamp pour cathéter selon l'une quelconque des revendications 1 à 5 ou la revendication 8, **caractérisé en ce que** la base (10') comprend dans la zone une formation de surface (60') par laquelle l'utilisateur tient le cathéter (2') pendant qu'il est en train d'être fixé avec la pince de serrage (25').

10. Clamp pour cathéter selon l'une quelconque des revendications précédentes, **caractérisé en ce que** la pince de serrage (25, 25') comprend un élément élastique (32) avec une gorge (33, 34) s'étendant à travers, et **en ce que** le cathéter (2, 2') s'étend le long de la gorge (33, 34).
